# EUROPÄISCHE PATENTSCHRIFT

(11) **EP 0 645 123 B1**
(45) Veröffentlichungstag und Bekanntmachung des Hinweises auf die Patenterteilung: **27.05.1998**
(21) Anmeldenummer: 94113487.6
(22) Anmeldetag: 30.08.1994
(51) Int. Cl.: A61C 5/06, A61M 5/31

(54) **Spritze zum dosierten Abgeben von viskosen, dentalen Werkstoffen**
Syringe for dosing viscous, dental substances
Seringue de dosage de substances visqueues dentaires

(30) Priorität: 23.09.1993 DE 4332310
(43) Veröffentlichungstag der Anmeldung: 29.03.1995
(73) Patentinhaber: HERAEUS KULZER GMBH, D-61273 Wehrheim (DE)
(72) Erfinder: Eykmann, Rudolf, D-61273 Wehrheim (DE); Fritze, Joachim, Dr., D-61381 Friedrichsdorf (DE); Uhrig, Birgit, D-61276 Neu-Anspach (DE); Schödel, Dieter, Dr., D-65193 Wiesbaden (DE)
(74) Vertreter: Grimm, Ekkehard, Dipl.-Phys.

(56) Entgegenhaltungen:
- EP-A- 0 319 639
- EP-A- 0 328 504
- EP-A- 0 379 177
- DE-A- 4 200 044
- US-A- 3 638 650
- US-A- 3 756 390

## Beschreibung

Die vorliegende Erfindung betrifft eine Spritze zum dosierten Abgeben von pastösen, dentalen Werkstoffen.

Eine solche Spritze ist beispielsweise aus der Produktinformation der Heraeus Kulzer GmbH "Charisma-Inlays - Gewinn durch Perfektion und Ästhetik" (31292/D 125 sK dt./WPR 12 12 200) bekannt. Diese Spritzen, in denen unter dem Produktnamen "Charisma" (Charisma ist eingetragenes Warenzeichen der Heraeus Kulzer GmbH) vertriebene, viskose Dentalwerkstoffe abgefüllt vertrieben werden, weisen eine Patrone auf, die den Dentalwerkstoff enthält. Diese Patrone ist an ihrem Austragsende im Außenumfang in Form einer Material-Austrittsdüse verjüngt ausgebildet, auf die eine Verschlußkappe aufgesetzt wird. In das dem Austragsende gegenüberliegende Ende der Patrone taucht ein Drehkolben ein, der sich an seinem Ende in einen hülsenförmigen Stopfen einlegt, der seinerseits gegen den in der Patrone abgefüllten viskosen Werkstoff anliegt. Der Drehkolben ist durchgehend mit einem Gewinde versehen, das in einem Lager in Form einer Mutter geführt ist. Um die Dentalwerkstoffe aus der Patrone auszudrücken, wird die Verschlußkappe der Patrone entfernt und der Drehkolben, der an seinem Ende ein Griffteil besitzt, in die Patrone hineingedreht, so daß der Stopfen zum Austrittsende der Patrone hin verschoben wird und den Werkstoff unter Druck setzt. Diese Spritzen haben sich im Einsatz über Jahre gut bewährt.

Eine weitere Spritze zum Austragen von viskosen Dentalwerkstoffen ist beispielsweise aus der US-PS 3,581,399 bekannt. Diese Spritze besitzt eine auswechselbare Spitze oder Düse, die beispielsweise in Form eines dünnen Kanals gebogen verläuft, um das Dentalmaterial gezielt an eine Stelle eines Zahns zu bringen, der bearbeitet werden soll. Diese Spitze wird auf das Ende der Patrone aufgeschraubt oder ist in einer anderen Ausführung aufklappbar verriegelt.

Die DE-A1 42 00 044 offenbart eine Dentalpatrone gemäß dem Oberbegriff von Anspruch 1, zum Ausgeben von Mehrkomponentenmaterial für Zahnfüllungen, die in eine sogenannte Hebelspritze eingesetzt wird. An dem Austragsende der Patrone ist eine Austragstülle fest verbunden.

Das DE-GM 78 37 177 gibt eine Spritze zur Direktapplikation von Zahnfüllungsmaterial an, wobei der das Füllungsmaterial aufnehmende Behälter als faltbare Kapsel mit Austragsdüse ausgebildet ist, die in die Patrone eingesetzt wird.

Aus der EP-A-0328504 ist es bekannt, daß eine einen Energierichtungsgeber für Ultraschallenergie aufweisende Material-Austrittdüse mittels Erwärmung durch Ultraschallenergie an der Kunststoff-Patrone einer Spritze befestigt wird.

Die US-PS 3,900,954 gibt eine Spritze an, die ähnlich der Spritze nach der US-PS 3,581,399 ist; in eine Patrone wird ein Kolben unter Reibung eingesetzt, der eine vorstehende Spitze aufweist, die sich in ein hülsenförmiges Teil drückt, in dem sich das Dentalmaterial befindet. Das kleine, hülsenförmige Teil ist an das Ende der Patrone angesetzt und bildet gleichzeitig die Austragsdüse für das Material.

Die angegebenen Patronen, die Dentalmaterialien aufnehmen, werden üblicherweise von einem hinteren Ende, d.h. einem Ende, das der Austragsdüse gegenüberliegt, befüllt. Nach dem Befüllen von hinten wird dann der eingangs beschriebene hülsenförmige Stopfen in das offene Befüllende der Patrone eingedrückt, bis er gegen das eingefüllte Material anliegt, gegen den dann später der Drehkolben zum Austragen des Materials drückt. Da solche Patronen, die das Dentalmaterial aufnehmen, auf der Innenseite eine geringe Konizität aufweisen, mit einem größeren Durchmesser auf der Seite, von der aus das Dentalmaterial eingefüllt und der Stopfen eingesetzt wird, die zum Entformen des Werkstücks nach dem Spritzguß notwendig ist, liegt der Stopfen mit unterschiedlicher Spannung in axialer Richtung der Patrone an deren Innenwand an. Gerade bei niedrig viskosen Materialien kann die Dichtigkeit der Patrone zu Problemen führen. Falls ein Stopfen verwendet wird, der eine höhere Dichtigkeit am Übergang zu der Innenwand der Patrone aufweist, entsteht beim Befüllvorgang der Patrone mit Material nach Einsetzen des Stopfens zwischen Material und Stopfen ein Luftpolster, das nicht entweichen kann. Um diesem Problem zu entgegnen, wurden solche Stopfen mit einem mittigen Loch versehen, wobei dann eine Antriebsspindel mit einem Dorn versehen wurde, um das Loch wieder zu verschließen. Falls bei einer solchen Ausführungsform nach einem Austrag von Material aus der Patrone der Stopfen entlastet werden soll, zieht sich zwangsläufig bei einem zu starken Zurückdrehen des Drehkolbens aus der Spritze der Dorn aus dem Loch des Stopfens heraus, so daß das in der Patrone befindliche Material der Umgebungsluft ausgesetzt wird. Hierbei kann es zu einer Aushärtung des Materials kommen, zumindest zu einer Änderung der Materialeigenschaften.

Gerade im Dentalbereich werden in solche Spritzen oder Applikationsinstrumente sehr unterschiedliche Materialien eingesetzt, z.B. auch solche, die unter Licht- oder Wärmeeinwirkung polymerisieren.

Ausgehend von dem vorstehend beschriebenen Stand der Technik liegt der vorliegenden Erfindung die Aufgabe zugrunde, eine Spritze zum dosierten Abgeben von Werkstoffen, insbesondere von dentalen Werkstoffen, anzugeben, die ein Befüllen der Spritze bzw. deren Patrone ermöglicht, ohne daß hierbei die Problematik eines Luftpolsters zwischen Dentalmaterial und Stopfen auftritt, und die darüberhinaus ein maschinelles Befüllen und Verschließen der Patrone erlaubt.

Die vorstehende Aufgabe wird durch eine Spritze wie in Anspruch 1 definiert, gelöst.

Eine solche Spritze oder Patrone wird nicht, wie dies beim Stand der Technik üblich ist, vom hinteren Ende aus, von dem aus der Drehkolben in die Patrone eintritt, befüllt, sondern von der Vorderseite, d.h. von dem Austragsende aus. Die Patrone ist ein hülsenförmiges Teil das über seine Länge einen etwa gleichen Querschnitt besitzt, mit einer geringen Konizität am Austragsende. Beim Befüllen der Patrone wird von dem austragsseitigen Ende der Patrone aus (das Ende, das dem Eintauchende des Drehkolbens gegenüberliegt) der Stopfen in die Patrone eingesetzt und geringfügig in die Patrone hineingeschoben. Anschließend wird der Stopfen beim Einfüllvorgang der Materialien, ggf. bis zu einem Anschlag der Patrone, nach hinten verschoben, wobei diese Verschiebung durch das an dem Stopfen anliegende Material bewirkt wird. Hierbei ist also kein Luftpolster zwischen Stopfen und Material vorhanden, zumal gerade zu Anfang des Füllvorgangs die Luft aus der Patrone im Bereich des Stopfens noch entweichen kann. Nach dem Befüllen der Patrone wird das Ende der Patrone, an dem eine Verbindungs- oder Ankoppelfläche vorgesehen ist, mit einer Material-Austrittsdüse durch Ultraschallschweißung verbunden. Diese Ultraschallschweißung hat den Vorteil, daß die Schallwellen gezielt in den Verbindungsbereich zwischen den Verbindungsflächen der Patrone bzw. der Austrittsdüse eingekoppelt werden können, so daß nur an dieser Stelle die Schallwellen in Wärme umgesetzt werden und demzufolge keine Wärme auf das in der Patrone befindliche Dentalmaterial übertragen wird. Eine solche Befüllung der Patronen mit Material bzw. das anschließende Verschließen mittels Ultraschallschweißen kann maschinell durchgeführt werden. Dabei ist auch von Vorteil, daß die Ultraschallwellen kaum hörbar sind und somit in der Umgebung der Abfüllmaschine mit einer solchen Ultraschallschweißeinrichtung keine Störgeräusche belästigend auftreten.

Eine solche Ultraschallschweißung erfolgt im 20 bis 40 kHz-Frequenzbereich, wobei dem höheren Frequenzbereich der Vorzug zu geben ist. In diesem hohen Frequenzbereich wird eine Ausgangsleistung des Schweißkopfs zwischen 350 und 700 Watt verwendet.

Bevorzugt sollte die mit der Patrone zu verbindende Austrittsdüse sowie die Patrone selbst aus Polypropylen bestehen. Bevorzugt wird die Verbindungsfläche der Material-Austrittsdüse parallel zu der Stirnseite der Patrone ausgerichtet. Hierdurch wird sichergestellt, daß die Schallwellen gleichmäßig um den Umfang der Patrone verteilt eingekoppelt werden; falls diese Verbindungsfläche geringfügig über den Außenumfang der Patrone vorsteht, ist diese ausreichend bei dem Ultraschallschweißen von dem in der Patrone befindlichen Material entfernt, außerdem kann eine gute Abkühlung der Schweißstelle nach dem Ultraschallschweißen erfolgen, da die Wärme nach außen abgeführt wird. Um die Ultraschallenergie gezielt im Bereich der Verbindungsfläche der Material-Austrittsdüse einkoppeln zu können, wird in einer vorteilhaften Ausgestaltung auf der Verbindungsfläche der Material-Austrittsdüse eine umlaufende Erhöhung ausgebildet, die einen Energierichtungsgeber bildet. Bevorzugt läuft dieser Energierichtungsgeber an seinem freien Ende spitz zu. In diesen Energierichtungsgeber wird, bevorzugt in dessen freies Ende bzw. in dessen Spitze, die Ultraschallenergie eingekoppelt, so daß sich dieser Energierichtungsgeber von der Spitze aus erwärmt, der Thermoplast fließt und sich mit der Patrone verbindet. Hierbei werden die Patrone und die Material-Austrittsdüse gegeneinander unter Druck gehalten.

Wie bereits vorstehend erwähnt wurde, sollte die Material-Austrittsdüse einen umlaufenden, über die Verbindungsfläche vorstehenden Rand aufweisen, der sich an der Außenseite der Patrone anlegt. Hierdurch wird neben einer guten Wärmeabführung auch eine große Dichtfläche gebildet, die sich einerseits in Richtung der Wand der Patrone erstreckt und andererseits eine geringe Strecke im Bereich des vorstehenden, überkragenden Rands der Austrittsdüse in Längsrichtung der Patrone verläuft. Hierbei wird der vorstehend erwähnte Energierichtungsgeber im Übergangsbereich zwischen Ankopplungsfläche und dem vorstehenden Rand angeordnet.

Um einen Ultraschallkopf exakt zu der Ankopplungsfläche beim Ultraschallschweißen ansetzen zu können, wird auf der Außenseite der Material-Austrittsdüse eine Außenfläche (Verbindungsfläche) vorgesehen, die etwa parallel zu der Verbindungsfläche verläuft. Auf dieser Außenfläche wird der Ultraschallkopf angesetzt.

Weitere Einzelheiten und Merkmale der Erfindung ergeben sich aus der nachfolgenden Beschreibung eines Ausführungsbeispiels anhand der Zeichnung. In der Zeichnung zeigt:
- Figur 1: eine seitliche Draufsicht auf eine Spritze mit einem Adapter und einer darin eingesetzten Patrone,
- Figur 2: eine Patrone mit verschiedenen Austrittsdüsen,
- Figur 3 und Figur 4: zwei verschiedene Austrittsdüsen in einer vergrößerten Darstellung und
- Figur 5: eine Austrittsdüse mit einer Verschlußkappe.

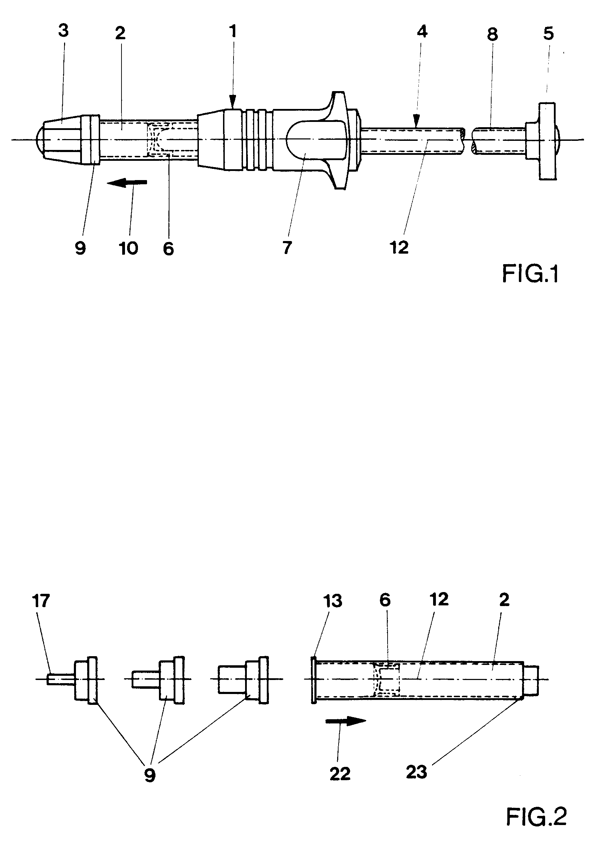

Eine Spritze zum dosierten Abgeben insbesondere von pastösen Werkstoffen, wie sie in Figur 1 dargestellt ist, weist einen Adapter 1 auf, in dem eine Patrone 2 gehalten ist, die an ihrem freien, aus dem Adapter 1 vorstehenden Ende mit einer Verschlußkappe 3 verschlossen ist. Die Patrone 2 ist mit einem auszutragenden, zu verarbeitenden Werkstoff befüllt. In den Adapter 1 tritt auf dem der Patrone 2 gegenüberliegenden Ende ein Drehkolben 4 ein, der einen Griff 5 an seinem freien Ende besitzt. Das Eintauchende des Drehkolbens 4 drückt gegen einen Stopfen 6, der in Figur 1 mit unterbrochenen Linien angedeutet ist. Der Drehkolben 4 wird in dem Adapterteil 1 in einem Lager 7 gehalten, in das er mit seinem Gewinde 8 eingeschraubt ist. Um Material aus der Patrone 2 über die Austrittsdüse 9, wie sie in verschiedenen Ausführungen in den Figuren 2 bis 5 gezeigt ist, auszutragen, wird der Drehkolben 4 am Griff 5 gedreht, so daß der Stopfen 6 in Richtung des Pfeils 10 in Figur 1 vorgeschoben wird und gegen das in der Patrone 2 eingefüllte Material drückt. Nach dem Gebrauch wird der Drehkolben 4 wieder geringfügig zurückgedreht, so daß der Stopfen 6 entlastet wird, und das Verschlußteil bzw. die Verschlußkappe 3 wird wieder auf die Austrittsdüse 9 aufgesetzt.

Je nachdem, welche Dentalmaterialien für welchen Verwendungszweck in die Patrone 2 eingefüllt werden, müssen, auch im Hinblick auf die verschiedenen Anwendungen, verschieden geformte Austrittsdüsen 9 an die Patrone 2 angesetzt werden. Hierzu können verschiedene Ausführungsformen der Austrittsdüse 9, wie sie in den Figuren 2 bis 5 dargestellt ist, mit der Patrone 2 verbunden werden.

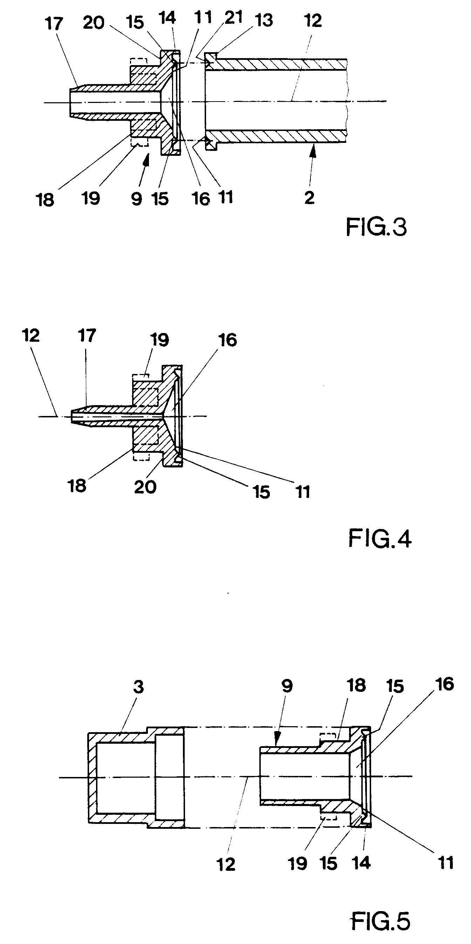

Die Verbindung der Austrittsdüse 9 mit der Patrone 2 erfolgt mittels Ultraschallschweißen. Hierzu sind die Verbindungsflächen 11 zwischen der Patrone 2 und der Austrittsdüse 9 plan und parallel zueinander verlaufend ausgebildet, wobei diese Flächen 11 jeweils senkrecht zu der Achse 12 der Patrone 2 bzw. der Austrittsdüse 9 verlaufen. Die Patrone 2 besitzt einen außen überkragenden Rand 13, um insbesondere die Verbindungsfläche 11 an der Patrone 2 zu vergrößern. Die Austrittsdüse 9 besitzt um den Umfang der Verbindungsfläche 11 umlaufend einen über die Ebene der Verbindungsfläche 11 vorstehenden Rand 14, wobei der Innendurchmesser des Bereichs, der von diesem Rand 14 umgeben wird, etwa dem Außenumfang der Patrone 2 im Bereich des überkragenden Rands 13 entspricht, so daß beim Zusammensetzen der Patrone 2 und der Autrittsdüse 9 der vorstehende Rand 14 den überkragenden Rand 13 außen übergreift. Auf der Verbindungsfläche 10 der Austrittsdüse 9 befindet sich ein umlaufender Vorsprung oder Energierichtungsgeber 15, der im Querschnitt, wie Figur 3 und 4 zeigt, dreieckförmig ausgebildet ist.

Die einzelnen Austrittsdüsen 9 weisen einen vergrößerten Einlaufbereich 16 auf, der trichterförmig ausgebildet ist und auf der einen Seite dem Innendurchmesser der Patrone 2 und auf der anderen Seite dem Innendurchmesser der Austragsspitze 17 angepaßt ist. Auf der Außenseite ist an jeder der verschiedenen, in den Figuren 2 bis 5 dargestellten Austrittsdüsen 9 ein Schulterbereich 18 in Form eines zylindrischen Abschnitts vorhanden, der in den einzelnen Ausführungsformen der Austrittsdüsen mit den unterschiedlich dimensionierten Austragsspitzen 17 jeweils denselben Außendurchmesser und dieselbe axiale Länge aufweist. Auf diesen Schulterbereich 18 der verschiedenen Austrittsdüsen 9 kann eine jeweils identische Verschlußkappe 3, wie es die Figur 1 zeigt, aufgesetzt werden; dieses kappenförmige Verschlußteil 3 kann an Vorsprüngen 19, die in den Figuren 3, 4 und 5 angedeutet sind, über nicht dargestellte Nuten und Vertiefungen in der Verschlußkappe 3 verriegelt werden kann.

Um die beiden Kunststoffteile, d.h. die Patrone 2 und die Austrittsdüse 9, miteinander zu verbinden, werden die beiden Teile entsprechend der Anordnung in den Figuren 2 und 3 zueinander ausgerichtet und mit ihren Verbindungsflächen 11 aufeinander gesetzt. Die beiden Ankoppelflächen 11 der Austrittsdüse 9 einerseits und der Austrittsdüse 9 andererseits werden in dieser Stellung durch den auf der Ankoppelfläche 11 der Patrone 2 umlaufenden Energierichtungsgeber 15 auf Abstand zueinander gehalten. In dieser Stellung wird auf die Außenseite der Austrittsdüse 9, parallel zu den Ankopplungflächen 11 verlaufend, ein Ultraschallkopf aufgesetzt. Hierzu sind die einzelnen Austrittsdüsen 9 mit einer Ankopplungsfläche 20 versehen, die durch die Stirnfläche eines zylindrischen Abschnitts der Austrittsdüse 9 gebildet ist, der in seinem Durchmesser größer als der Durchmesser des zylindrischen Schulterbereichs ist. Über diese Ankopplungsfläche 20 kann dann Ultraschallenergie, d.h. Ultraschall mit einer Frequenz zwi schen 20 bis 40 kHz mit einer Leistung von etwa 350 bis 700 Watt, gezielt eingekoppelt werden, und zwar in die Spitze des Energierichtungsgebers 15, der dadurch erwärmt wird und fließt, so daß die beiden Teile an ihren Verbindungsflächen 11 durch den weichen bzw. verflüssigten Kunststoff verklebt werden. Um die Verbindung der beiden Teile noch zu verbessern bzw. die Verbindungsfläche noch zu vergrößern, kann in der Verbindungsfläche 11 der Patrone 2 eine umlaufende Nut 21 ausgebildet sein, wie sie in Figur 3 gezeigt ist, die im Querschnit z.B. eine dreieckige Form aufweist, die dem Querschnitt des Energierichtungsgebers 15 angepaßt ist. Diese umlaufende Nut 21 ist allerdings so dimensioniert, daß ihr Querschnitt etwas geringer als der Querschnitt des Energierichtungsgebers 15 ist, damit keine Hohlräume beim Verschweißen der beiden Teile miteinander verbleiben.

Vorzugsweise wird die Austrittsdüse 9 und/oder die Patrone 2 aus Polypropylen hergestellt. Dieses Material zeichnet sich dadurch aus, daß es gut schweißbar und schlecht wärmeleitend ist. Die Festigkeit der Schweißstelle erreicht annähernd die Eigenfestigkeit des Materials. Die Verschleißdauer ist gering und eine Belastung des Materials ist ausgeschlossen.

Die Spritze bzw. Patrone 2, die mit der Austrittsdüse 9 verschlossen wird, hat den Vorteil, daß sie von vorne, d.h. von dem Ende aus, das auf die Austrittsdüse 9 aufgesetzt wird, herstellerseitig mit Material befüllt werden kann. Hierzu wird zunächst in die leere Patrone 2 der Stopfen 6 eingesetzt, der sich dichtend an die Innenwand der Patrone 2 anlegt. Anschließend wird die Patrone 2 in eine Befüllstation eingesetzt und der Werkstoff in die Patrone 2 eingedrückt, wobei sich der Stopfen 6 zum hinteren Ende der Patrone 2 in Richtung des Pfeils 22 in Figur 2 verschiebt, bis der Stopfen an einem in seinem Durchmesser verringerten Anlagebereich 23 anliegt. Bei diesem Befüllvorgang treten keine Lufteinschlüsse im Bereich des Stopfens 6 auf, da vor dem Stopfen befindliche Luft bereits in der Anfangsphase des Befüllvorgangs mit Material aus der Patrone 2 austritt. Anschließend wird die befüllte Patrone 2 in eine Ultraschallschweißeinrichtung eingesetzt und mit der Austrittsdüse 9 verschweißt. Das Ultraschallschweißen hat den Vorteil, daß nur der umlaufende Vorsprung bzw. der Energierichtungsgeber 15 auf der Verbindungsfläche 11 der Patrone 2 erwärmt wird.

Es hat sich gezeigt, daß das Material, das beim Ultraschallschweißen bereits in der Patrone 2 eingefüllt ist, keiner Erwärmung unterliegt bzw. durch den Ultraschallschweißvorgang in keiner Weise beeinflußt wird.

## Patentansprüche

1. Spritze zum dosierten Abgeben von pastösen, dentalen Werkstoffen, mit einer den Werkstoff aufnehmenden, hülsenförmigen Patrone (2) aus Kunststoff, in die von deren einem Ende aus ein Drehkolben (4) eintaucht und die an ihrem anderen Ende eine Material-Austrittsdüse (9) aufweist, die wenigstens an ihrem der Patrone zugekehrten Ende aus Kunststoff ausgebildet ist, und die eine gegenüber dem Innendurchmesser der Patrone geringere Öffnungsweite an ihrem der Patrone abgekehrten Ende besitzt, wobei der Drehkolben an seinem Eintauchende gegen einen Stopfen drückt und in einem Lager (7) verschiebbar gehalten ist, dadurch gekennzeichnet, daß zur Befestigung der Material-Austrittsdüse an der von ihrem Austragsende aus mit pastösem dentalem Werkstoff befüllten Patrone diese über ihre Länge einen etwa gleichen Querschnitt aufweist und an ihrem Austragsende zur Bildung ihrer Verbindungsfläche mit der Material-Austrittsdüse einen ihren Außenumfang überkragenden Rand (13) besitzt, wobei die Verbindungsfläche (11) am Rand (13) einer Verbindungsfläche (11) an der Material-Austrittsdüse (9) gegenüberliegt, und daß von der Verbindungsfläche (11) der Material-Austrittsdüse (9) ein umlaufender, der Verbindungsfläche (11) der Patrone (2) zugekehrter Energierichtungsgeber (15) für Ultraschallenergie vorspringt, über den nach Erwärmung durch Ultraschallenergie die Patrone (2) an der Material-Austrittsdüse (9) befestigt ist.

2. Spritze nach Anspruch 1, dadurch gekennzeichnet, daß die Verbindungsfläche (11) der Material-Austrittsdüse (9) parallel zu der Stirnseite der Patrone (2) verläuft.

3. Spritze nach Anspruch 1 oder 2, dadurch gekennzeichnet, daß auf der Ankoppelfläche (11) der Material-Austrittsdüse (9) eine umlaufende Erhöhung ausgebildet ist, die den Energierichtungsgeber (15) bildet.

4. Spritze nach Anspruch 3, dadurch gekennzeichnet, daß der Energierichtungsgeber (15) an seinem freien Ende spitz zuläuft.

5. Spritze nach einem der Ansprüche 1 bis 4, dadurch gekennzeichnet, daß die Material-Austrittsdüse (9) einen umlaufenden, über die Ankoppelfläche vorstehenden Rand (14) aufweist, der sich an die Außenseite der Patrone (2) anlegt.

6. Spritze nach Anspruch 5 in Verbindung mit Anspruch 3 oder 4, dadurch gekennzeichnet, daß der Energierichtungsgeber (15) im Übergangsbereich zwischen Ankoppelfläche (11) und dem vorstehenden Rand (14) angeordnet ist.

7. Spritze nach einem der Ansprüche 1 bis 6, dadurch gekennzeichnet, daß die Material-Austrittsdüse (9) auf ihrer Außenseite eine Ankopplungsfläche (20) aufweist, die etwa parallel zu der Verbindungsfläche (11) verläuft.

8. Spritze nach einem der Ansprüche 1 bis 7, dadurch gekennzeichnet, daß die Patrone (2) und die Material-Austrittsdüse (9) aus Polypropylen bestehen.

## Claims

1. A syringe for the controlled discharge of pasty, dental materials, said syringe comprising a sleeve-shaped cartridge (2) of plastics which holds the material and into which a rotary piston (4) is inserted from the one end of said cartridge and which at its other end comprises a material discharge nozzle (9) which at least at its end facing the cartridge is made of plastics and which at its end facing away from the cartridge has an opening width which is smaller than the inside diameter of the cartridge, with the rotary piston being pushed at its insertion end against a stopper and being displaceably held in a bearing (7), characterized in that for fastening the material discharge nozzle to the cartridge filled from its discharge end with pasty, dental material, said cartridge has about the same cross section over its length and has its discharge end provided with an edge (13) projecting over the outside circumference thereof for forming the connecting surface of the cartridge with the material discharge nozzle, with the connecting surface (11) being opposite the material discharge nozzle (9) at the edge (13) of a connecting surface (11), and that an encircling energy flow indicator (15) which faces the connecting surface (11) of the cartridge (2) and is intended for ultrasonic energy projects from the connecting surface (11) of the material discharge nozzle (9), with the cartridge (2) being fastened to the material discharge nozzle (9) via said indicator after heating by ultrasonic energy.

2. A syringe according to claim 1, characterized in that the connecting surface (11) of the material discharge nozzle (9) runs parallel to the front side of the cartridge (2).

3. A syringe according to claim 1 or 2, characterized in that a raised encircling portion which forms the energy flow indicator (15) is formed on the connecting surface (11) of the material discharge nozzle (9).

4. A syringe according to claim 3, characterized in that the energy flow indicator (15) tapers to a point at its free end.

5. A syringe according to any one of claims 1 to 4, characterized in that the material discharge nozzle (9) has an encircling edge (14) which projects beyond the connecting surface and is in contact with an outside of the cartridge (2).

6. A syringe according to claim 5 in combination with claim 3 or 4, characterized in that the energy flow indicator (15) is located in a transitional region between the connecting surface (11) and the projecting edge (14).

7. A syringe according to any one of claims 1 to 6, characterized in that the material discharge nozzle (9) is provided on its outside with a connecting surface (20) which is approximately parallel to the connecting surface (11).

8. A syringe according to any one of claims 1 to 7, characterized in that the cartridge (2) and the material discharge nozzle (9) are made of polypropylene.

## Revendications

1. Seringue pour la distribution dosée de substances pâteuses dentaires, comportant une cartouche (2) en matière plastique recevant la substance et en forme de douille, dans laquelle plonge depuis l'une de ses extrémités un piston tournant (4) et qui présente à son autre extrémité une buse de sortie de substance (9) qui est réalisée en matière plastique au moins à son extrémité orientée vers la cartouche, et qui présente une largeur d'ouverture inférieure au diamètre intérieur de la cartouche à son extrémité détournée de la cartouche, le piston tournant s'appuyant par son extrémité de plongée contre un bouchon et étant retenu de façon mobile dans un palier (7), caractérisée en ce que pour fixer la buse de sortie de substance sur la cartouche remplie depuis son extrémité de sortie avec une substance pâteuse dentaire, ladite cartouche présente une section transversale approximativement égale sur sa longueur et pour former sa surface de jonction avec la buse de sortie de substance elle possède à son extrémité de sortie une bordure (13) en saillie par rapport à sa périphérie extérieure, la surface de jonction (11) se trouvant, au niveau de la bordure (13), en face d'une surface de jonction (11) sur la buse de sortie de substance (9), et en ce qu'un générateur directionnel d'énergie (15) pour de l'énergie ultrasonore, périphérique et orienté vers la surface de jonction (11) de la cartouche (2), fait saillie depuis la surface de jonction (11) de la buse de sortie de substance (9), via lequel la cartouche (2) est fixée sur la buse de sortie de substance (9) après échauffement par l'énergie ultrasonore.

2. Seringue selon la revendication 1, caractérisée en ce que la surface de jonction (11) de la buse de sortie de substance (9) s'étend parallèlement à la face frontale de la cartouche (2).

3. Seringue selon l'une ou l'autre des revendications 1 et 2, caractérisée en ce qu'une élévation périphérique est réalisée sur la surface de couplage (11) de la buse de sortie de substance (9), qui forme le générateur directionnel d'énergie (15).

4. Seringue selon la revendication 3, caractérisée en ce que le générateur directionnel d'énergie (15) converge en pointe à son extrémité libre.

5. Seringue selon l'une quelconque des revendications 1 à 4, caractérisée en ce que la buse de sortie de substance (9) présente une bordure périphérique (14) qui dépasse au-delà de la surface de couplage et qui vient en appui contre la face extérieure de la cartouche (2).

6. Seringue selon la revendication 5 en dépendance de l'une ou l'autre des revendications 3 et 4, caractérisée en ce que le générateur directionnel d'énergie (15) est agencé dans la zone de transition entre la surface de couplage (11) et la bordure en saillie (14).

7. Seringue selon l'une quelconque des revendications 1 à 6, caractérisée en ce que la buse de sortie de substance (9) présente sur sa face extérieure une surface de couplage (20) qui s'étend approximativement parallèlement à la surface de jonction (11).

8. Seringue selon l'une quelconque des revendications 1 à 7, caractérisée en ce que la cartouche (2) et la buse de sortie de substance (9) sont constituées en polypropylène.
